# EUROPEAN PATENT APPLICATION

(11) **EP 2 730 278 A1**
(43) Date of publication of application: **14.05.2014**
(21) Application number: 12191890.8
(22) Date of filing: 08.11.2012
(51) Int. Cl.: A61K 9/14, A61K 9/20, A61K 9/48, A61K 31/4035

(54) **Composition melt**

(71) Applicant: Ratiopharm GmbH, 89079 Ulm (DE)
(72) Inventor: Holfinger, Konstantin, 81379 München (DE); Brück, Sandra, 81477 München (DE)
(74) Representative: Gallagher, Kirk James

(57) **Abstract**

The present invention relates to compositions comprising compound (I) and processes for the preparation thereof.

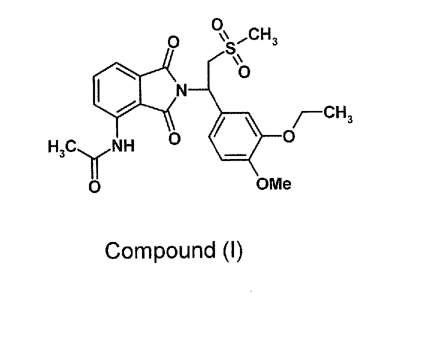

## Description

### Field of the Invention

The present invention relates to N-[2-[1-(3-ethoxy-4-methoxyphenyl)-2-(methylsulfonyl)ethyl]-1,3-dioxo-2,3-dihydro-1H-isoindol-4-yl]acetamide (compound (I)). In particular, the invention relates to compositions comprising compound (I) and processes for the preparation thereof.

### Background of the Invention

N-[2-[1-(3-ethoxy-4-methoxyphenyl)-2-(methylsulfonyl)ethyl]-1,3-dioxo-2,3-dihydro-1H-isoindol-4-yl]acetamide is an experimental inhibitor of phosphodiesterase (PDE) 4, interferon gamma antagonist; IL-2 gene inhibitor; leukotriene synthesis inhibitor; TNF alpha synthesis inhibitor; IL-6 antagonist; IL-17 gene inhibitor; angiogenesis inhibitor; NO-synthase inhibitor; and IL-23 gene inhibitor.

The structure of N-[2-[1-(3-ethoxy-4-methoxyphenyl)-2-(methylsulfonyl)ethyl]-1,3-dioxo-2,3-dihydro-1H-isoindol-4-yl]acetamide is shown below:

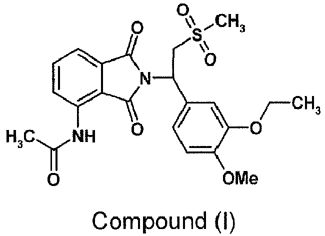

Enantiomerically pure compound (I) has low solubility in aqueous solution. In aqueous buffer pH 7.4 the solubility measured at room temperature is about 0.01mg/ml. Poor solubility typically leads to poor oral bioavailability, fed/fasted variations in bioavailability, cumbersome and inconvenient dosage forms, and may necessitate the use of harsh solubilising agents that are associated with adverse side effects.

WO2011/059931 describes a method for preparing a nanosuspension of a poorly soluble drug in order to improve bioavailability. Said method comprises stirring the drug, which has been micronized, in an aqueous polymeric excipient solution in the absence of surfactants and passing the concentrate through a high-shear microfluidizer processor to obtain the nanosuspension.

WO2009/120167 describes solid forms of (+)-N-[2-[1-(3-ethoxy-4-methoxyphenyl)-2-(methylsulfonyl)ethyl]-1,3-dioxo-2,3-dihydro-1H-isoindol-4-yl]acetamide (also known as apremilast). However, there is no enabling disclosure of an amorphous form.

There remains a need in the art for solid forms of compound (I) with improved properties.

### Summary of the Invention

In a first aspect, the present invention relates to a process for preparing a composition comprising compound (I), the process comprising melting compound (I), together with at least one suitable excipient.

In a second aspect, the present invention relates to a composition comprising compound (I) obtainable by a process according to the first aspect.

In a third aspect, the present invention relates to a melt comprising compound (I).

In a fourth aspect, the present invention relates to a pharmaceutical composition comprising a composition according the second aspect, or a melt according to the third aspect, and a pharmaceutically acceptable excipient.

In a fifth aspect, the present invention relates to an amorphous form of compound (I).

In a sixth aspect, the present invention relates to a pharmaceutical composition comprising an amorphous form of compound (I) and a pharmaceutically acceptable excipient.

In a seventh aspect, the present invention relates to a method of treating or preventing a disease or disorder ameliorated by the inhibition of TNF-[alpha] production, wherein the method comprises administering a therapeutically or prophylactically effective amount of an amorphous form of compound (I) according to the fifth aspect, a melt according to any one of the second and third aspects, or a pharmaceutical composition according to any one of the fourth and sixth aspects.

In a eighth aspect; the present invention relates to a method of treating or preventing a disease or disorder ameliorated by the inhibition of PDE4, wherein the method comprises administering a therapeutically or prophylactically effective amount of an amorphous form of compound (I) according to the fifth aspect, a melt according to any one of the second and third aspects, or a pharmaceutical composition according to any one of the fourth and sixth aspects.

In a ninth aspect, the present invention relates to a method of treating or preventing a cancer, wherein the method comprises administering a therapeutically or prophylactically effective amount of an amorphous form of compound (I) according to the fifth aspect, a melt according to any one of the second and third aspects, or a pharmaceutical composition according to any one of the fourth and sixth aspects.

Surprisingly, it has been found that the compositions of the invention have advantageous properties over compositions comprising crystalline forms of compound (I). In particular, dissolution studies have shown that a composition comprising a melt of compound (I) exhibits a faster rate of dissolution over a composition comprising crystalline compound (I). Furthermore, compositions comprising a melt of compound (I) have been shown to retain at least 80% dissolution within 30 minutes in dissolution tests after being subjected to accelerated storage conditions.

### Brief Description of the Figures

Figure 1 shows the dissolution profile of immediate release tablets comprising a melt of apremilast (example 1) in various buffers.
Figure 2 shows the initial dissolution profile of immediate release tablets comprising a melt of apremilast (example 1) compared to the dissolution profile of the tablets after they have been stored for 12 weeks at 40°C/75% relative humidity.
Figure 3 shows the XRPD pattern for the tablets of example 1.
Figure 4 shows the initial dissolution profile of immediate release capsules comprising a melt of apremilast (example 2) compared to the dissolution profile of the capsules after they have been stored for 8 weeks at 40°C/75% relative humidity.
Figure 5 shows the XRPD pattern for the capsule formulation of example 2.
Figure 6 shows the initial dissolution profile of immediate release tablets comprising crystalline apremilast (example 3) compared to the dissolution profile of the tablets after they have been stored for 12 weeks at 40°C/75% relative humidity.
Figure 7 shows the initial dissolution profile of immediate release capsules comprising crystalline apremilast (example 4) compared to the dissolution profile of the capsules after they have been stored for 8 weeks at 40°C/75% relative humidity.

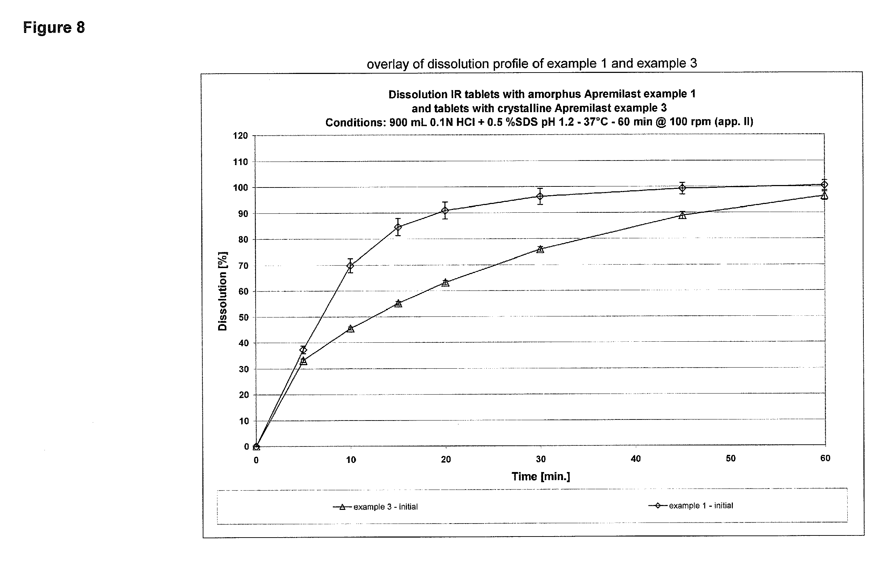

Figure 8 shows the dissolution profile of the apremilast tablets of example 1 compared to that of the apremilast tablets of example 3.

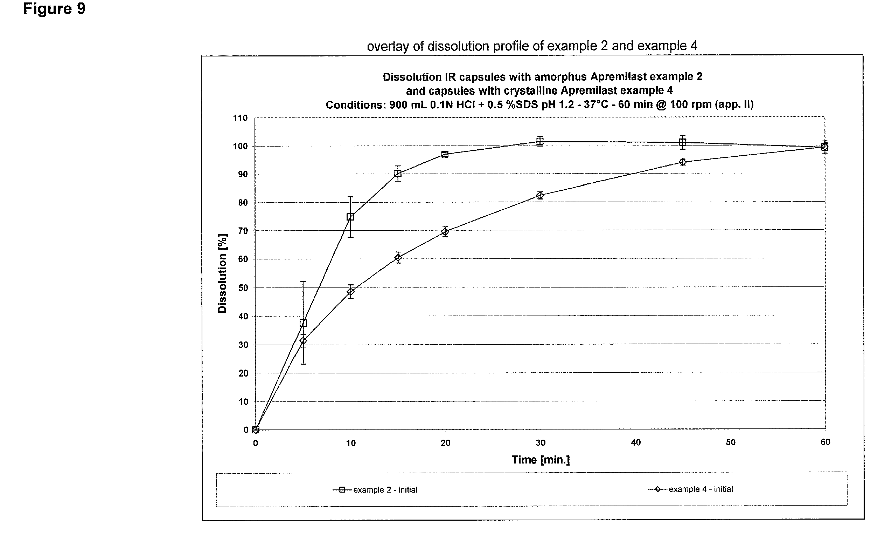

Figure 9 shows the dissolution profile of the apremilast capsules of example 2 compared to that of the apremilast capsules of example 4.
Figure 10 shows the XRPD pattern of a melt of ampremilast and Kollidon VA 64 and citric acid (1:1:1) after storage for 12 weeks at 25°C/60% relative humidity.
Figure 11 shows the XRPD pattern of crystalline Form B of ampremilast.

### Detailed Description of the Invention

### Definitions

As used herein the term "apremilast" refers to (+)-N-[2-[1-(3-ethoxy-4-methoxyphenyl)-2-(methylsulfonyl)ethyl]-1,3-dioxo-2,3-dihydro-1H-isoindol-4-yl]acetamide (compound (Ia))

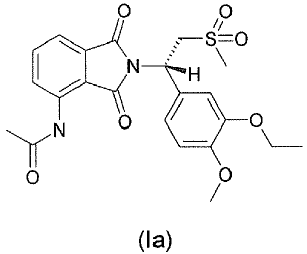

As used herein the term "a melt" refers to a molecular dispersion of at least two components which results after said components are melted together.

In all aspects of the present invention previously discussed, the invention includes, where appropriate all enantiomers of compound (I). The person skilled in the art will recognise compounds that possess optical properties (one or more chiral carbon atoms). The corresponding enantiomers may be isolated/prepared by methods known in the art. Thus, where reference is made to compound (I), both the (+) and (-)-enantiomers of compound (I) separately, or mixtures thereof in any ratio, including a racemic mixture of enantiomers for example, are encompassed.

In one embodiment compound (I) is substantially all in the (+)-enantiomeric form and is also known as apremilast; preferably compound (I) is 80% or more in the (+)-enantiomeric form, more preferably compound (I) is 90% or more in the (+)-enantiomeric form, more preferably compound (I) is 95% or more in the (+)-enantiomeric form, more preferably compound (I) is 99% or more in the (+)-enantiomeric form.

### Processes

In a first aspect, the present invention relates to a process for preparing a composition comprising compound (I), the process comprising melting compound (I) together with at least one suitable excipient.

Preferably, the process of the first aspect is for preparing a composition comprising amorphous compound (I).

The product of melting compound (I) together with at least one suitable excipient may be referred to as a melt comprising compound (I).

In one embodiment compound (I) is melted together with one to five suitable excipients. In another embodiment compound (I) is melted together with one to three suitable excipients. Preferably, compound (I) is melted together with one suitable excipient.

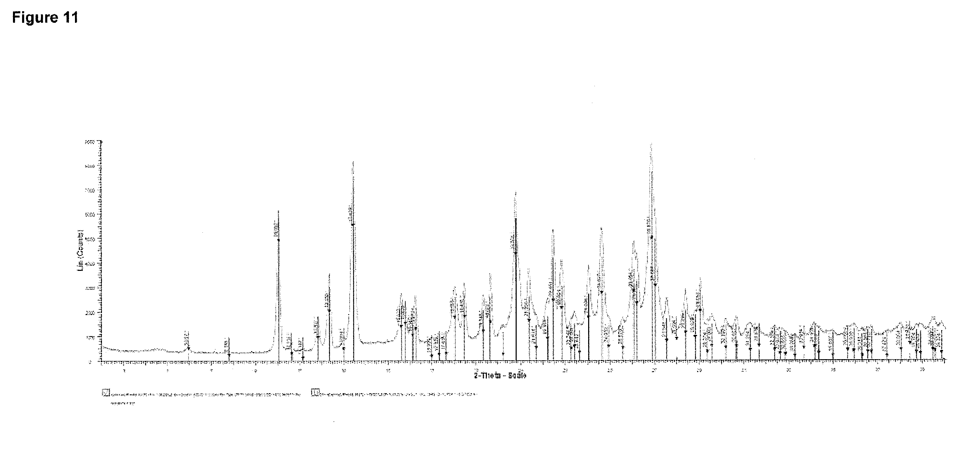

The starting form of compound (I) which is melted together with the suitable excipient can be any form of compound (I). In one embodiment the starting form of compound (I) is crystalline Form B. Preferably, the starting form of compound (I) is Form B with a purity of 90% or more; more preferably 95% or more; most preferably 99% or more. Form B may be characterised by XRPD peaks located at 10.1, 13.5, 20.7, 22.5, 24.7 and 26.9 degrees 2theta. Form B may be further characterised by XRPD peaks located at 10.1, 12.4, 13.5, 15.7, 16.3, 18.1, 20.7, 22.5, 24.7, 26.2, 26.9, and 29.1 degrees 2theta. The XRPD of Form B is provided in Figure 11. Form B can be prepared according to the procedure of WO2000/025777, which is incorporated herein by reference.

In one embodiment the suitable excipient has a melting point of 50°C or more and/or a glass transition temperature of 15°C or more.

In another embodiment the suitable excipient has a melting point of 60°C or more, more preferably 70°C or more, more preferably 80°C or more, more preferably 90°C or more, more preferably 100°C or more.

In another embodiment the suitable excipient has a melting point of about 50°C to about 400°C, more preferably about 50°C to about 300°C.

In one embodiment the suitable excipient has a glass transition temperature of 20°C or more, more preferably 25°C or more, more preferably 30°C or more, more preferably 35°C or more, more preferably 40°C or more.

In another embodiment the suitable excipient has a glass transition temperature of about 15°C to about 200°C, preferably about 15°C to about 100°C.

The melting point and the glass transition temperature can be determined by any known means. For example, the melting point and glass transition temperature can be determined by means of differential scanning calorimetry (DSC). Suitable methods of determining melting points and glass transition temperatures are as described in the European Pharmacopoeia 7 (Ph. Eur., chapter 2.2.34) and the United States Pharmacopoeia (USP, chapter 891), the content of which is incorporated herein by reference.

In one embodiment the suitable excipient is selected from the group consisting of a polymer, a copolymer, a saccharide, an oligosaccharide, a polysaccharide, a sugar alcohol, a lipid, and a wax.

In one embodiment the suitable excipient is a polymer.

In one embodiment the polymer is selected from the group consisting of cellulose derivatives, such as hydroxypropylmethylcellulose (HPMC), carboxymethylcellulose (CMC), ethylcellulose, methylcellulose, hydroxyethylcellulose, ethylhydroxyethylcellulose, and hydroxypropylcellulose (HPC), micro-crystalline cellulose, starch, arabic gum, tragacanth gum, guar gum, alginic acid, alginates, polyvinylpyrrolidone (PVP), polyvinylacetates (PVAC), polyvinyl alcohols (PVA), polyvinyl alcohol derivatives, polymers of the acrylic acid and its salts, polyacrylamides, polymethacrylates, polymethacrylate derivatives, vinylpyrrolidone vinylacetate copolymers (copovidone), Soluton, polyalkylene glycoles, such as poly(propylene glycol) and polyethylene glycol and its derivatives such as polyethylene glycol glycerides and fatty acid esters of polyethylene glycol, co-blockpolymers of the polyethylene glycol, in particular co-blockpolymers of polyethylene glycol and poly(propylene glycol), co-blockpolymers of ethylene oxide and propylene oxide (Poloxamer, Pluronic), sucrose fatty acid esters as well as mixtures of two or more of the mentioned polymers.

Preferably the polymer is copovidone.

In another embodiment the suitable excipient is a sugar alcohol.

In one embodiment the sugar alcohol is selected from the group consisting of lactose, mannitol, sorbitol, xylitol, isomalt, glucose, fructose, maltose, arabinose, and mixtures thereof.

The ratio of compound (I) to suitable excipient is not particularly limited. The ratio can be adjusted to obtain the desired dilution effect or to allow for the solubility of the active substance in the suitable excipient. For example, the weight ratio of compound (I) to excipient may be in the range of 1:1 to 1:1,000. Preferred ranges are for example 1:1 to 1:500, 1:1 to 1:100, 1:10 to 1:50, and in particular about 1:50. More preferably, the ratio is about 1:1. All of the above-mentioned upper and lower limits can also be combined with each other in order to form additional preferred ranges.

In one embodiment the temperature at which compound (I) and the at least one suitable excipient are melted at is between about 50°C and about 300°C; preferably between about 100°C and about 250°C, more preferably between about 150°C and about 250°C, more preferably about 200°C.

In one embodiment, the process further comprises extrusion of the melt. Extrusion can be carried out using any technique known in the art. For example, suitable techniques include those described in "Pharmaceutical Extrusion Technology" by Isaac Ghebre-Sellassie, Charles Martin, ISBN 0824740505, Informa Healthcare Verlag, 2003, the content of which is incorporated herein by reference.

In one embodiment the process further comprises cooling the melt. Cooling can be carried out by any known method in the art. Preferably, the melt is allowed to cool passively to room temperature.

In one embodiment the process further comprises reducing the particle size of the melt, for example by crushing, grinding etc. Particle size reduction can be carried out by any known method in the art.

### Compositions of Compound (I)

In a second aspect, the present invention relates to a composition comprising compound (I) obtainable by a process according to the first aspect. Preferably, the composition comprises amorphous compound (I).

In a third aspect, the present invention relates to a melt comprising compound (I).

Preferably, the melt comprises amorphous compound (I).

In one embodiment the melt comprises, in addition to compound (I), one to five suitable excipients; preferably the melt comprises one to three suitable excipients; more preferably the melt comprises one suitable excipient. In one embodiment the melt essentially consists of amorphous compound (I) and one suitable excipient. In one embodiment the melt consists of amorphous compound (I) and one suitable excipient.

In one embodiment, the amorphous form of compound (I) is substantially free of any crystalline forms. In another embodiment, the amorphous form of compound (I) contains 20% or less of any crystalline form, preferably 10% or less, more preferably 5% or less, more preferably 2% or less, more preferably 1 % or less.

In one embodiment the suitable excipient has a melting point of 50°C or more and/or a glass transition temperature of 15°C or more.

In another embodiment the suitable excipient has a melting point of 60°C or more, more preferably 70°C or more, more preferably 80°C or more, more preferably 90°C or more, more preferably 100°C or more.

In another embodiment the suitable excipient has a melting point of about 50°C to about 400°C, more preferably about 50°C to about 300°C.

In one embodiment the suitable excipient has a glass transition temperature of 20°C or more, more preferably 25°C or more, more preferably 30°C or more, more preferably 35°C or more, more preferably 40°C or more.

In another embodiment the suitable excipient has a glass transition temperature of about 15°C to about 200°C, preferably about 15°C to about 100°C.

The melting point and the glass transition temperature can be determined by any known means. For example, the melting point and glass transition temperature can be determined by means of differential scanning calorimetry (DSC). Suitable methods of determining melting points and glass transition temperatures are as described herein.

In one embodiment the suitable excipient is selected from the group consisting of a polymer, a copolymer, a saccharide, an oligosaccharide, a polysaccharide, a sugar alcohol, a lipid, and a wax.

In one embodiment the suitable excipient is a polymer.

In one embodiment the polymer is selected from the group consisting of cellulose derivatives, such as hydroxypropylmethylcellulose (HPMC), carboxymethylcellulose (CMC), ethylcellulose, methylcellulose, hydroxyethylcellulose, ethylhydroxyethylcellulose, and hydroxypropylcellulose (HPC), micro-crystalline cellulose, starch, arabic gum, tragacanth gum, guar gum, alginic acid, alginates, polyvinylpyrrolidone (PVP), polyvinylacetates (PVAC), polyvinyl alcohols (PVA), polyvinyl alcohol derivatives, polymers of the acrylic acid and its salts, polyacrylamides, polymethacrylates, polymethacrylate derivatives, vinylpyrrolidone vinylacetate copolymers (copovidone), Soluton, polyalkylene glycoles, such as poly(propylene glycol) and polyethylene glycol and its derivatives such as polyethylene glycol glycerides and fatty acid esters of polyethylene glycol, co-blockpolymers of the polyethylene glycol, in particular co-blockpolymers of polyethylene glycol and poly(propylene glycol), co-blockpolymers of ethylene oxide and propylene oxide (Poloxamer, Pluronic), sucrose fatty acid esters as well as mixtures of two or more of the mentioned polymers.

Preferably the polymer is copovidone.

In another embodiment the suitable excipient is a sugar alcohol.

In one embodiment the sugar alcohol is selected from the group consisting of lactose, mannitol, sorbitol, xylitol, isomalt, glucose, fructose, maltose, arabinose, and mixtures thereof.

The ratio of compound (I) to suitable excipient in the melt is not particularly limited. For example, the weight ratio of compound (I) to excipient may be in the range of 1:1 to 1:1,000. Preferred ranges are for example 1:1 to 1:500, 1:1 to 1:100, 1:10 to 1:50, and in particular about 1:50. More preferably, the ratio is about 1:1. All of the above-mentioned upper and lower limits can also be combined with each other in order to form additional preferred ranges.

In one embodiment, the melt is extruded. Extrusion can be carried out using any technique known in the art. For example, suitable techniques include those as referred to herein.

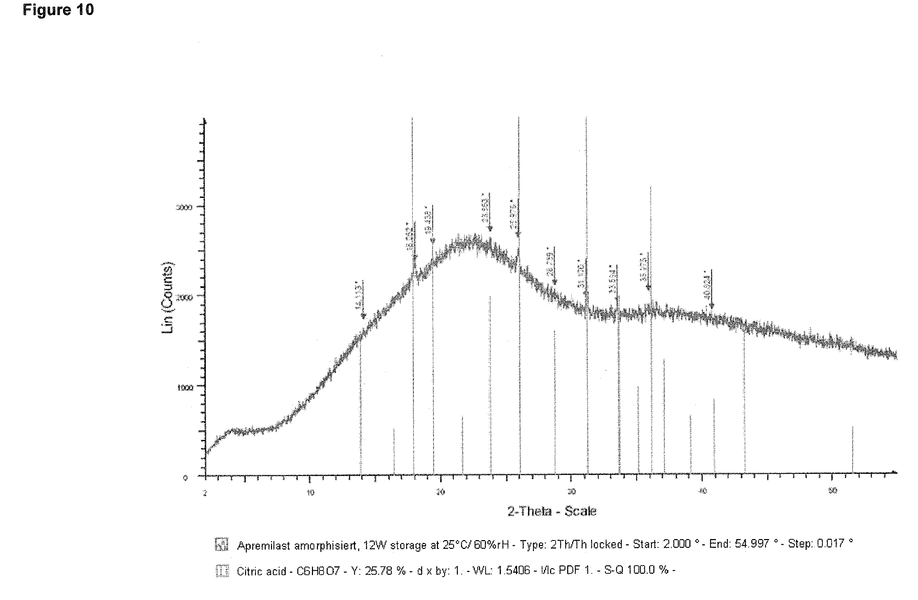

In one embodiment the melt comprises compound (I), copovidone and citric acid. In another embodiment the melt essentially consists of compound (I), copovidone and citric acid. In another embodiment the melt consists of compound (I), copovidone and citric acid. Preferably the ratio of compound (I):copovidone:citric acid is 1:1:1. In one embodiment the melt has an XPRD as depicted in Figure 10.

In a fifth aspect, the present invention relates to an amorphous form of compound (I).

In one embodiment, the amorphous form of compound (I) is substantially free of any crystalline forms. In another embodiment, the amorphous form of compound (I) contains 20% or less of any crystalline form, preferably 10% or less, more preferably 5% or less, more preferably 2% or less, more preferably 1% or less.

### Pharmaceutical Compositions

In a fourth aspect, the present invention relates to a pharmaceutical composition comprising a composition according the second aspect, or a melt according to the third aspect, and a pharmaceutically acceptable excipient.

In a sixth aspect, the present invention relates to a pharmaceutical composition comprising an amorphous form of compound (I) and a pharmaceutically acceptable excipient.

Pharmaceutical compositions may be prepared as medicaments to be administered orally, parenterally, rectally, transdermally, buccally, or nasally. Suitable forms for oral administration include tablets, compressed or coated pills, dragees, sachets, hard or gelatin capsules, sub-lingual tablets, syrups, and suspensions. Suitable forms of parenteral administration include an aqueous or non-aqueous solution or emulsion, while for rectal administration, suitable forms for administration include suppositories with hydrophilic or hydrophobic vehicle. For topical administration, the invention provides suitable transdermal delivery systems known in the art, and for nasal delivery, there are provided suitable aerosol delivery systems known in the art.

In addition to the active ingredient(s), the pharmaceutical compositions of the present invention may contain one or more excipients or adjuvants. Selection of excipients and the amounts to use may be readily determined by the formulation scientist based upon experience and consideration of standard procedures and reference works in the field.

Diluents increase the bulk of a solid pharmaceutical composition, and may make a pharmaceutical dosage form containing the composition easier for the patient and care giver to handle. Diluents for solid compositions include, for example, microcrystalline cellulose (e.g. Avicel®), microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit®), potassium chloride, powdered cellulose, sodium chloride, sorbitol, and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel®), hydroxypropyl methyl cellulose (e.g. Methocel®), liquid glucose, magnesium aluminum silicate, maltodextrin, methylcellulose, polymethacrylates, povidone (e.g. Kollidon®, Plasdone®), pregelatinized starch, sodium alginate, and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. Ac-Di-Sol®, Primellose®), colloidal silicon dioxide, croscarmellose sodium, crospovidone (e.g. Kollidon®, Polyplasdone®), guar gum, magnesium aluminum silicate, methyl cellulose, microcrystalline cellulose, polacrilin potassium, powdered cellulose, pregelatinized starch, sodium alginate, sodium starch glycolate (e.g. Explotab®), and starch.

Glidants can be added to improve the flowability of a non-compacted solid composition and to improve the accuracy of dosing. Excipients that may function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and die. Some excipients and active ingredients have a tendency to adhere to the surfaces of the punch and die, which can cause the product to have pitting and other surface irregularities. A lubricant can be added to the composition to reduce adhesion and ease the release of the product from the die. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavouring agents and flavour enhancers make the dosage form more palatable to the patient. Common flavouring agents and flavour enhancers for pharmaceutical products that may be included in the composition of the present invention include maltol, vanillin, ethyl vanillin, menthol, citric acid, fumaric acid, ethyl maltol, and tartaric acid.

Solid and liquid compositions may also be dried using any pharmaceutically acceptable colorant to improve their appearance and/or facilitate patient identification of the product and unit dosage level.

In liquid pharmaceutical compositions of the present invention, the active ingredient and any other solid excipients are suspended in a liquid carrier such as water, vegetable oil, alcohol, polyethylene glycol, propylene glycol or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol, and cetyl alcohol.

Liquid pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth, and xanthan gum.

Sweetening agents such as sorbitol, saccharin, sodium saccharin, sucrose, aspartame, fructose, mannitol, and invert sugar may be added to improve the taste.

Preservatives and chelating agents such as alcohol, sodium benzoate, butylated hydroxy toluene, butylated hydroxyanisole, and ethylenediamine tetraacetic acid may be added at levels safe for ingestion to improve storage stability.

According to the present invention, a liquid composition may also contain a buffer such as gluconic acid, lactic acid, citric acid or acetic acid, sodium gluconate, sodium lactate, sodium citrate, or sodium acetate.

Selection of excipients and the amounts used may be readily determined by the formulation scientist based upon experience and consideration of standard procedures and reference works in the field.

The solid compositions of the present invention include powders, granulates, aggregates, and compacted compositions. The dosages include dosages suitable for oral, buccal, rectal, parenteral (including subcutaneous, intramuscular, and intravenous), inhalant, and ophthalmic administration. Although the most suitable administration in any given case will depend on the nature and severity of the condition being treated, the most preferred route of the present invention is oral. The dosages may be conveniently presented in unit dosage form and prepared by any of the methods well known in the pharmaceutical arts.

Dosage forms include solid dosage forms like tablets, powders, capsules, suppositories, sachets, troches, and lozenges, as well as liquid syrups, suspensions, and elixirs.

The dosage form of the present invention may be a capsule containing the composition, preferably a powdered or granulated solid composition of the invention, within either a hard or soft shell. The shell may be made from gelatin, and, optionally, contain a plasticizer such as glycerin and sorbitol, and an opacifying agent or colorant.

The active ingredient and excipients may be formulated into compositions and dosage forms according to methods known in the art.

A composition for tableting or capsule filling can be prepared by wet granulation. In wet granulation, some or all of the active ingredients and excipients in powder form are blended, and then further mixed in the presence of a liquid, typically water, that causes the powders to clump into granules. The granulate is screened and/or milled, dried, and then screened and/or milled to the desired particle size. The granulate may then be tableted or other excipients may be added prior to tableting, such as a glidant and/or a lubricant.

A tableting composition can be prepared conventionally by dry blending. For example, the blended composition of the actives and excipients may be compacted into a slug or a sheet, and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention may comprise any of the aforementioned blends and granulates that were described with reference to tableting, however, they are not subjected to a final tableting step.

### Administration

Typically, a physician will determine the actual dosage which will be most suitable for an individual subject and it will vary with the age, weight and response of the particular patient and severity of the condition. The dosages below are exemplary of the average case. There can, of course, be individual instances where higher or lower dosage ranges are merited.

The compositions (or component parts thereof) of the present invention may be administered orally. In addition or in the alternative the compositions (or component parts thereof) of the present invention may be administered by direct injection. In addition or in the alternative the compositions (or component parts thereof) of the present invention may be administered topically. In addition or in the alternative the compositions (or component parts thereof) of the present invention may be administered by inhalation. In addition or in the alternative the compositions (or component parts thereof) of the present invention may also be administered by one or more of: parenteral, mucosal, intramuscular, intravenous, subcutaneous, intraocular or transdermal administration means, and are formulated for such administration.

By way of further example, the pharmaceutical composition of the present invention may be administered in accordance with a regimen of 1 to 10 times per day, such as once or twice per day. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The term "administered" also includes but is not limited to delivery by a mucosal route, for example, as a nasal spray or aerosol for inhalation or as an ingestible solution; a parenteral route where delivery is by an injectable form, such as, for example, an intravenous, intramuscular or subcutaneous route.

Hence, the pharmaceutical composition of the present invention may be administered by one or more of the following routes: oral administration, injection (such as direct injection), topical, inhalation, parenteral administration, mucosal administration, intramuscular administration, intravenous administration, subcutaneous administration, intraocular administration or transdermal administration.

### Medical uses

In a seventh aspect, the present invention relates to a method of treating or preventing a disease or disorder ameliorated by the inhibition of TNF-[alpha] production, wherein the method comprises administering a therapeutically or prophylactically effective amount of an amorphous form of compound (I) according to the fifth aspect, a melt according to any one of the second and third aspects, or a pharmaceutical composition according to any one of the fourth and sixth aspects.

In one embodiment the disease or disorder ameliorated by the inhibition of TNF-[alpha] production is selected from psoriasis; psoriatic arthritis; rheumatoid arthritis; chronic cutaneous sarcoid; giant cell arteritis; Parkinson's Disease; prurigo nodularis; lichen planus; complex apthosis; Behcet's Disease; lupus; hepatitis; uveitis; Sjogren's Disease; depression; interstitial cystitis; vulvodynia; prostatitis; osteoarthritis; diffuse large B cell lymphoma; polymysoitis; dermatomyositis; inclusion body myositis; erosive osteoarthritis; interstitial cystitis; hepatitis; endometriosis; radiculopathy; and pyoderma gangrenosum.

Alternatively, the seventh aspect relates to an amorphous form of compound (I) according to the fifth aspect, a melt of compound (I) according to the second and third aspects, or a pharmaceutical composition according to the fourth and sixth aspects of the invention are for use in the treatment of any of following: psoriasis; psoriatic arthritis; rheumatoid arthritis; chronic cutaneous sarcoid; giant cell arteritis; Parkinson's Disease; prurigo nodularis; lichen planus; complex apthosis; Behcet's Disease; lupus; hepatitis; uveitis; Sjogren's Disease; depression; interstitial cystitis; vulvodynia; prostatitis; osteoarthritis; diffuse large B cell lymphoma; polymysoitis; dermatomyositis; inclusiuon body myositis; erosive osteoarthritis; interstitial cystitis; hepatitis; endometriosis; radiculopathy; and pyoderma gangrenosum.

Alternatively, the seventh aspect relates to the use of an amorphous form of compound (I) according to the fifth aspect, a melt of compound (I) according to the second and third aspects, or a pharmaceutical composition according to the fourth and sixth aspects of the invention in the manufacture of a medicament for the treatment of any of following: psoriasis; psoriatic arthritis; rheumatoid arthritis; chronic cutaneous sarcoid; giant cell arteritis; Parkinson's Disease; prurigo nodularis; lichen planus; complex apthosis; Behcet's Disease; lupus; hepatitis; uveitis; Sjogren's Disease; depression; interstitial cystitis; vulvodynia; prostatitis; osteoarthritis; diffuse large B cell lymphoma; polymysoitis; dermatomyositis; inclusion body myositis; erosive osteoarthritis; interstitial cystitis; hepatitis; endometriosis; radiculopathy; and pyoderma gangrenosum.

In a eighth aspect, the present invention relates to a method of treating or preventing a disease or disorder ameliorated by the inhibition of PDE4, wherein the method comprises administering a therapeutically or prophylactically effective amount of an amorphous form of compound (I) according to the fifth aspect, a melt according to any one of the second and third aspects, or a pharmaceutical composition according to any one of the fourth and sixth aspects.

In one embodiment the disease or disorder ameliorated by the inhibition of of PDE4 is selected from HIV; hepatitis; adult respiratory distress syndrome; bone resorption diseases; chronic obstructive pulmonary diseases; chronic pulmonary inflammatory diseases; dermatitis; inflammatory skin disease, atopic dermatitis, cystic fibrosis; septic shock; sepsis; endotoxic shock; hemodynamic shock; sepsis syndrome; post ischemic reperfusion injury; meningitis; psoriasis; fibrotic disease; cachexia; graft rejection including graft versus host disease; auto immune disease; rheumatoid spondylitis; arthritic conditions, such as rheumatoid arthritis and osteoarthritis; osteoporosis; Crohn's disease; ulcerative colitis; inflammatory bowel disease; multiple sclerosis; systemic lupus erythrematosus; erythema nodosum leprosum in leprosy; radiation damage; asthma; and hyperoxic alveolar injury.

Alternatively, the eight aspect relates to an amorphous form of compound (I) according to the fifth aspect, a melt of compound (I) according to the second and third aspects, or a pharmaceutical composition according to the fourth and sixth aspects of the invention are for use in the treatment of any of following: HIV; hepatitis; adult respiratory distress syndrome; bone resorption diseases; chronic obstructive pulmonary diseases; chronic pulmonary inflammatory diseases; dermatitis; inflammatory skin disease, atopic dermatitis, cystic fibrosis; septic shock; sepsis; endotoxic shock; hemodynamic shock; sepsis syndrome; post ischemic reperfusion injury; meningitis; psoriasis; fibrotic disease; cachexia; graft rejection including graft versus host disease; auto immune disease; rheumatoid spondylitis; arthritic conditions, such as rheumatoid arthritis and osteoarthritis; osteoporosis; Crohn's disease; ulcerative colitis; inflammatory bowel disease; multiple sclerosis; systemic lupus erythrematosus; erythema nodosum leprosum in leprosy; radiation damage; asthma; and hyperoxic alveolar injury.

Alternatively, the eight aspect relates to the use of an amorphous form of compound (I) according to the fifth aspect, a melt of compound (I) according to the second and third aspects, or a pharmaceutical composition according to the fourth and sixth aspects of the invention in the manufacture of a medicament for the treatment of any of following: HIV; hepatitis; adult respiratory distress syndrome; bone resorption diseases; chronic obstructive pulmonary diseases; chronic pulmonary inflammatory diseases; dermatitis; inflammatory skin disease, atopic dermatitis, cystic fibrosis; septic shock; sepsis; endotoxic shock; hemodynamic shock; sepsis syndrome; post ischemic reperfusion injury; meningitis; psoriasis; fibrotic disease; cachexia; graft rejection including graft versus host disease; auto immune disease; rheumatoid spondylitis; arthritic conditions, such as rheumatoid arthritis and osteoarthritis; osteoporosis; Crohn's disease; ulcerative colitis; inflammatory bowel disease; multiple sclerosis; systemic lupus erythrematosus; erythema nodosum leprosum in leprosy; radiation damage; asthma; and hyperoxic alveolar injury.

In an ninth aspect, the present invention relates to a method of treating or preventing a cancer, wherein the method comprises administering a therapeutically or prophylactically effective amount of an amorphous form of compound (I) according to the fifth aspect, a melt according to any one of the second and third aspects, or a pharmaceutical composition according to any one of the fourth and sixth aspects.

Alternatively, the ninth aspect relates to an amorphous form of compound (I) according to the fifth aspect, a melt of compound (I) according to the second and third aspects, or a pharmaceutical composition according to the fourth and sixth aspects of the invention are for use in the treatment of cancer.

Alternatively, the ninth aspect relates to the use of an amorphous form of compound (I) according to the fifth aspect, a melt of compound (I) according to the second and third aspects, or a pharmaceutical composition according to the fourth and sixth aspects of the invention in the preparation of a medicament for the treatment of cancer.

In one embodiment the cancer is selected from multiple myeloma, malignant melanoma, malignant glioma, leukaemia and a solid tumour.

Although the foregoing compositions and methods have been described in some detail by way of illustration and examples for purposes of clarity of understanding, it will be apparent to those skilled in the art that certain changes and modifications may be made. Therefore, the description should not be construed as limiting the scope of the invention, which is delineated by the appended claims.

### Examples

### Materials and Methods

XPRD patterns were generated on a Bruker-AXS D8 Advance powder X-ray diffractometer (Bruker-AXS, Karlsruhe, Germany), equipped with a Vantec-1 detector (3° opening angle). The sample holder was rotated in a plane parallel to its surface at 20 rpm during measurement. The measurement conditions were as follows: Radiation: Cu K-alpha, Source 38 kV / 40 mA, divergence slit (variable V6, antiscattering slit 5.59 mm, detector slit 10.28 mm, start angle 2°, end angle 55°, Step 0.016° 2Θ).

DSC measurements were performed using a Mettler Toledo DSC 1 apparatus. The work was performed at a heating rate of 1 to 20°C/min, preferably 5 to 15°C/min, and at a cooling rate of 5 to 25°C/min, preferably 10 to 20°C/min.

Dissolution conditions were 900 mL buffer and 0.5% SDS, 37°C, 100 rpm paddle (USP app.II). The preparation of the buffers employed is provided below:

For pH of approximately 1.2 weigh 150 g of HCl 25% into a 10 litre vessel. Fill up with purified water to 10kg.

For sodium acetate trihydrate pH 4.5 (50 mM) buffer weigh about 29.9 g of CH₃COONa x 3H₂O into a 10 litre vessel. Fill up with purified water to 10kg. Adjust the pH with CH₃COOH to 4.5.

For potassium phosphate, pH 6.8 (50 mM) buffer weigh 68.05 g of KH₂PO₄ x H₂O and 9.6 g of NaOH pellets into a 10 litre vessel. Fill up with purified water to 10kg. Adjust the pH with NaOH or H₃PO₄ to 6.8.

### Example 1 (Immediate release tablets comprising a melt of apremilast)

| **Composition** | **Functionality** | **mg/tablet** |
|---|---|---|
| Apremilast | active ingredient | 20.00 |
| Copovidone (*Kollidon*^{®} *VA 64*) | polymer | 20.00 |
| Combination of microcrystalline cellulose, SiO₂, Sodium starch glycolate and Sodium stearyl Fumarate (Prosolv® EASYtab) | All-in-One Composite (binder/filler, glidant, desintegrant, lubricant) | 180,00 |

### Manufacturing:

Kollidon VA64 and apremilast were melted on a heating plate at a temperature of 200°C. After cooling and solidification the material was crushed with mortar and pestle. Prosolv^{®} EASYtab was added to the melt, sieved over 630 µm and blended for 15 minutes in a tumble mixer (e.g. Turbula^{®} T10B). The final blend was compressed to 7 mm round tablets on a rotary tablet press Riva^{®} Piccola with a hardness of approximately 100 N.

### Dissolution testing of tablets:

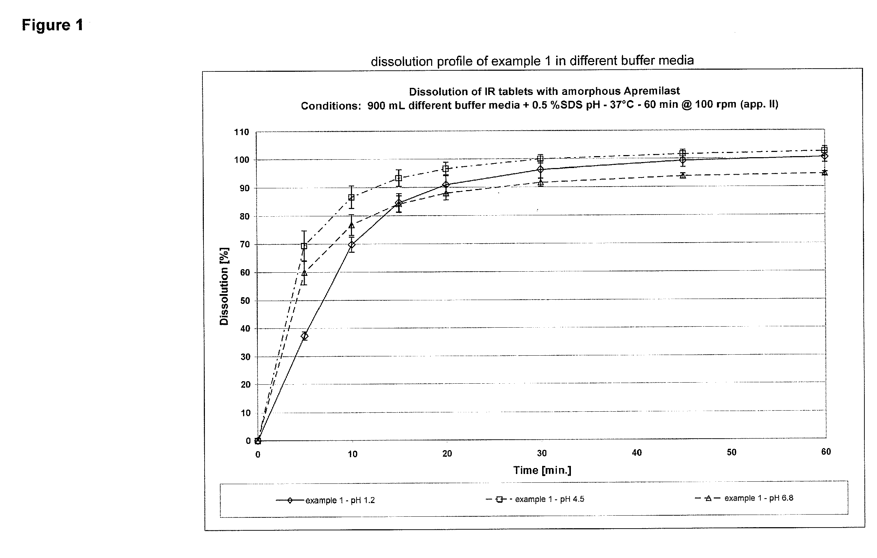

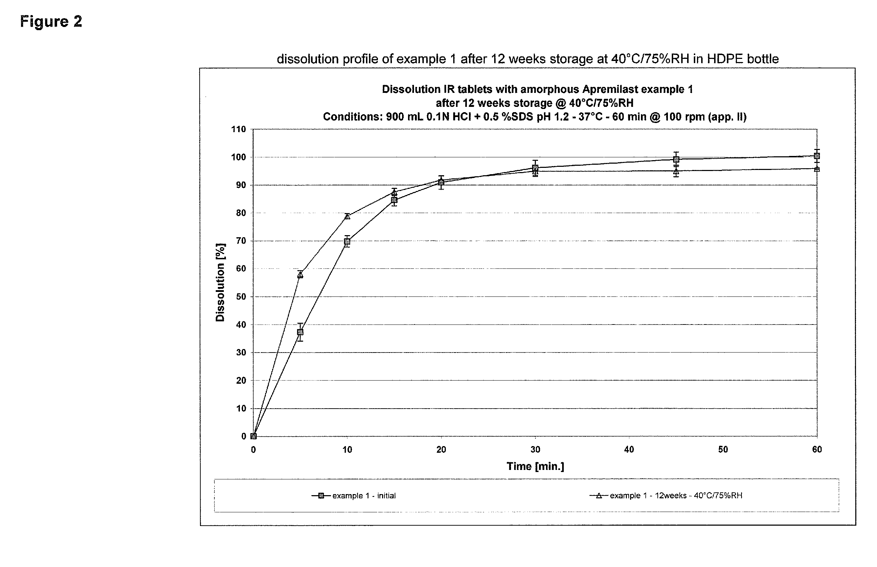

The dissolution testing was carried out using different buffer media. The profiles are shown in the Figure 1. Figure 2 shows the dissolution profile after 12 weeks accelerated storage conditions at 40°C/75% relative humidity.

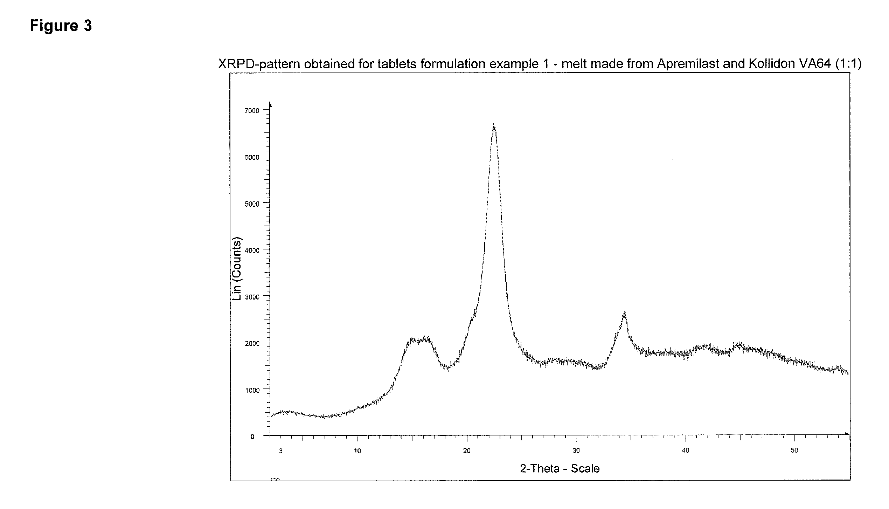

A sample of example 1 was analyzed on a Bruker-AXS D8 Advance powder X-ray diffractometer. A completely amorphous halo pattern was obtained, showing the profile of microcrystalline cellulose (i.e. Avicel PH102) (see Figure 3).

### Example 2 (Immediate release capsules comprising a melt of apremilast)

| **Composition** | **Functionality** | **mg/capsule** |
|---|---|---|
| Apremilast | active ingredient | 20.00 |
| Copovidone (*Kollidon^{®} VA 64*) | polymer | 20.00 |
| StarCap 1500 (corn starch and pregelatinized starch) | filler/disintegrant | 180,00 |

### Manufacturing:

The Kollidon VA64 and apremilast were melted on a heating plate. After solidification the material were crushed with mortar and pestle. StarCap 1500 was added and mixed with the melt. Capsules size 2 were filled.

### Dissolution testing of capsules:

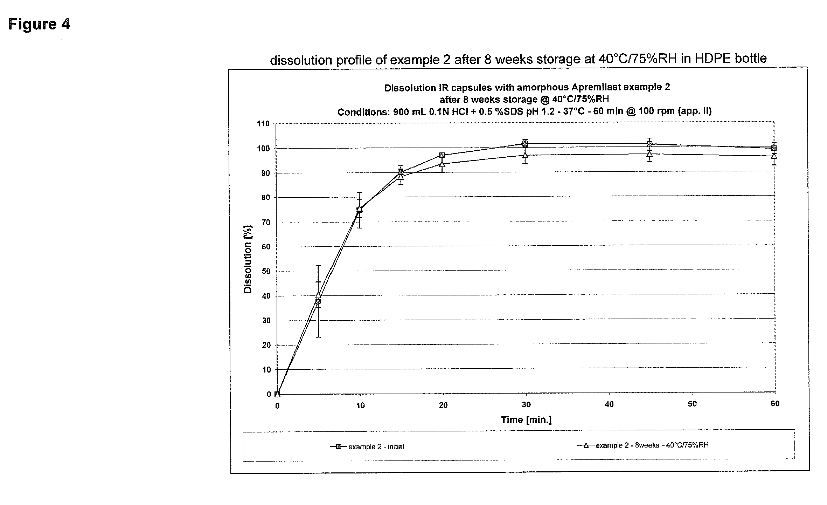

Figure 4 shows the dissolution profile after 8 weeks accelerated storage conditions at 40°C/75% relative humidity.

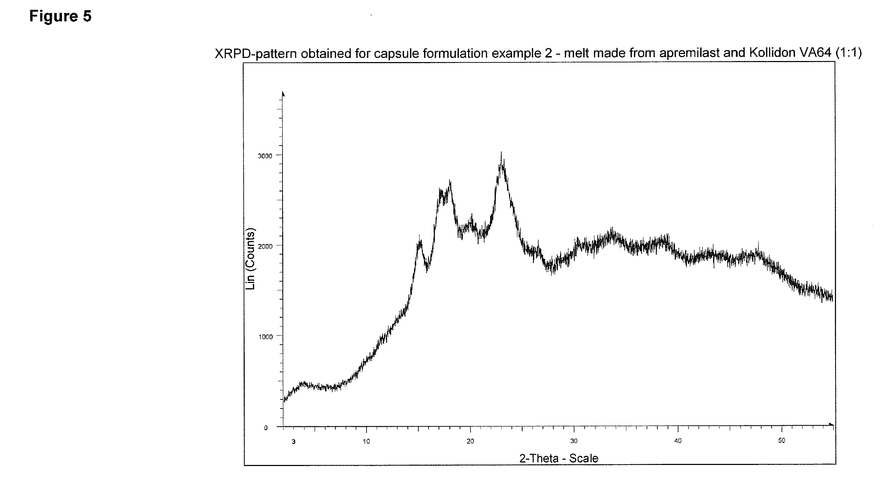

A sample of example 2 was analyzed on a Bruker-AXS D8 Advance powder X-ray diffractometer. A completely amorphous halo pattern was obtained (see Figure 5).

### Reference Example 3 (Immediate release tablets comprising crystalline apremilast)

| **Composition** | **Functionality** | **mg/tablet** |
|---|---|---|
| Apremilast | active ingredient | 20.00 |
| Agglomerated Lactose | filler/ binder | 147.00 |
| Acdisol | super disintegrant | 6.70 |
| Microcrystalline cellulose | filler | 44.50 |
| Magnesium stearate | lubricant | 2.20 |

### Manufacturing

The mixture of excipients was sieved over a 500 µm sieve and blended for 10 min. Apremilast was milled with mortar and pestle and added to this mixture. The final blend was sieved over a 800 µm sieve and blended for 5 minutes. Round tablets 7 mm were compressed on a rotary tablet press, Riva piccolo. The tablets were stored for 12 weeks at 40°C/75%.

### Dissolution testing of tablets:

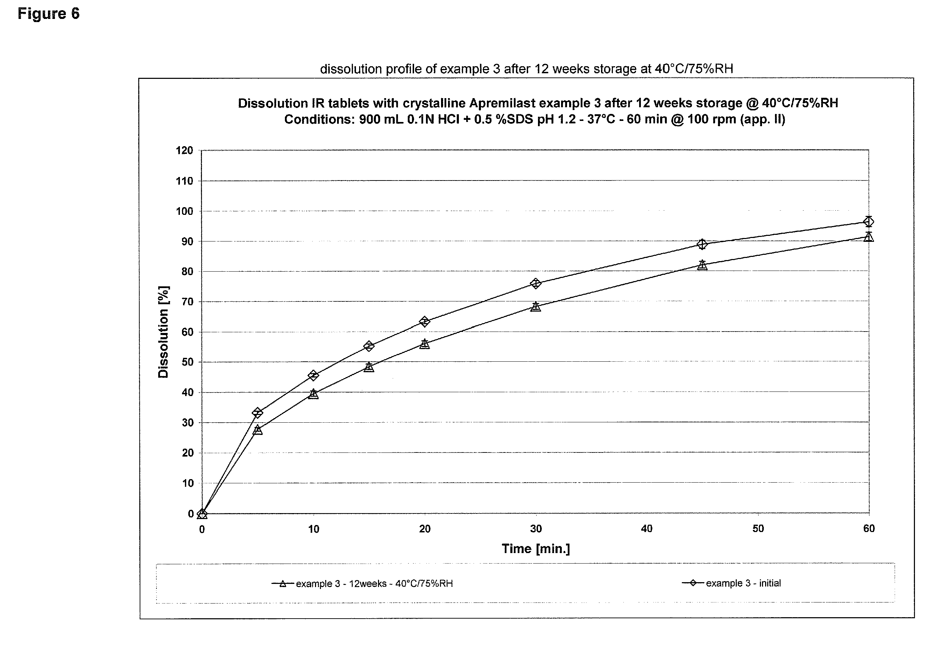

The dissolution profiles of the tablets initially and after accelerated storage are shown in the Figure 6.

### Reference Example 4 (Immediate release capsules comprising crystalline apremilast)

| **Composition** | **Functionality** | **mg/capsule** |
|---|---|---|
| Apremilast | active ingredient | 20.00 |
| Agglomerated Lactose | filler/ binder | 147.00 |
| Acdisol | super disintegrant | 6.70 |
| Microcrystalline cellulose | filler | 44.50 |
| Magnesium stearate | lubricant | 2.20 |

### Manufacturing:

The mixture of excipients was sieved over a 500 µm sieve and blended for 10 minutes. Apremilast was milled with mortar and pestle and added to this mixture. The mixture was blended for 5 minutes and was filled into capsules size 2. The capsules were stored for 8 weeks at 40°C/75% relative humidity.

### Dissolution testing of capsules:

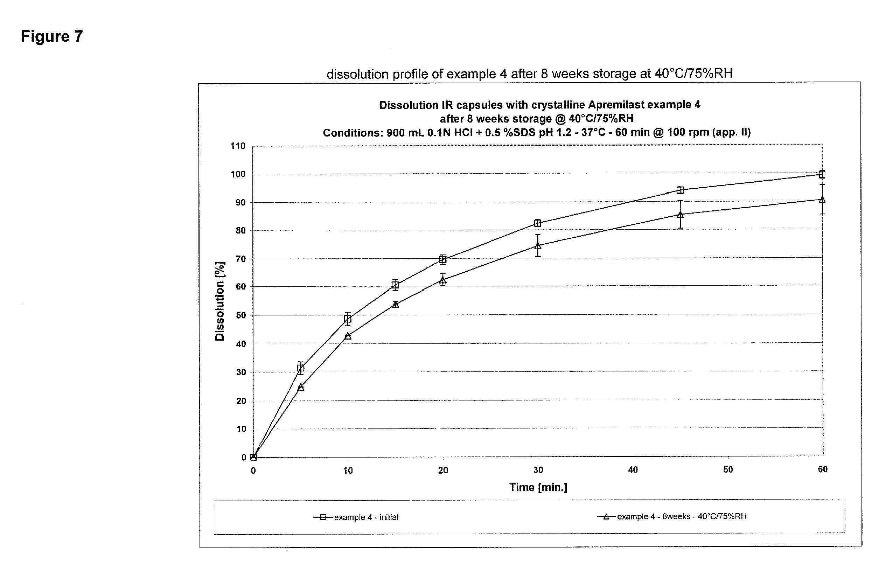

The dissolution profiles of the tablets initially and after accelerated storage are shown in the Figure 7.

The invention will now be described by means of the following numbered paragraphs:
1. A process for preparing a composition comprising compound (I) which process comprises melting compound (I), together with at least one suitable excipient. 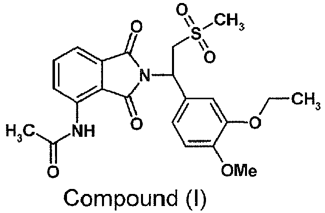
2. A process according to paragraph 1 wherein the composition comprises amorphous compound (I).
3. A process according to any preceding paragraph wherein the suitable excipient has a melting point of 50°C or more and/or a glass transition temperature of 15°C or more.
4. A process according to any preceding paragraph wherein the suitable excipient is selected from a polymer, a copolymer, a saccharide, an oligosaccharide, a polysaccharide, a sugar alcohol, a lipid, and a wax;
5. A process according to any preceding paragraph wherein the suitable excipient is a polymer.
6. A process according to paragraph 5 wherein the polymer is selected from the group consisting of cellulose derivatives, such as hydroxypropylmethylcellulose (HPMC), carboxymethylcellulose (CMC), ethylcellulose, methylcellulose, hydroxyethylcellulose, ethylhydroxyethylcellulose, and hydroxypropylcellulose (HPC), micro-crystalline cellulose, starch, arabic gum, tragacanth gum, guar gum, alginic acid, alginates, polyvinylpyrrolidone (PVP), polyvinylacetates (PVAC), polyvinyl alcohols (PVA), polyvinyl alcohol derivatives, polymers of the acrylic acid and its salts, polyacrylamides, polymethacrylates, polymethacrylate derivatives, vinylpyrrolidone vinylacetate copolymers (copovidone), Soluton, polyalkylene glycoles, such as poly(propylene glycol) and polyethylene glycol and its derivatives such as polyethylene glycol glycerides and fatty acid esters of polyethylene glycol, co-blockpolymers of the polyethylene glycol, in particular co-block polymers of polyethylene glycol and poly(propylene glycol), co-block polymers of ethylene oxide and propylene oxide (Poloxamer, Pluronic), sucrose fatty acid esters as well as mixtures of two or more of the mentioned polymers.
7. A process according to any of paragraphs 4 to 6 wherein the polymer is copovidone.
8. A process according to any preceding paragraph wherein compound (I) and the suitable excipient are melted at a temperature between about 50°C and about 300°C; preferably between 100°C and 250°C, more preferably between 150°C and 250°C.
9. A process according to any preceding paragraph further comprising extruding the product of melting compound (I), together with at least one suitable excipient.
10. A composition comprising compound (I) obtainable by a process according to any preceding paragraph.
11. A melt comprising compound (I).
12. A melt according to paragraph 11 wherein compound (I) is in an amorphous form.
13. A pharmaceutical composition comprising a composition according to claim 10, or a melt according to any one of paragraphs 11 and 12, and a pharmaceutically acceptable excipient.
14. A pharmaceutical composition comprising an amorphous form of compound (I) and a pharmaceutically acceptable excipient.
15. A method of treating or preventing a disease or disorder ameliorated by the inhibition of TNF-[alpha] production, wherein the method comprises administering a therapeutically or prophylactically effective amount of a melt according to any one of paragraphs 10 and 11, or a pharmaceutical composition according to any one of paragraphs 13 and 14.
16. The method of paragraph 15 wherein the disease or disorder is selected from psoriasis; psoriatic arthritis; rheumatoid arthritis; chronic cutaneous sarcoid; giant cell arteritis; Parkinson's Disease; prurigo nodularis; lichen planus; complex apthosis; Behcet's Disease; lupus; hepatitis; uveitis; Sjogren's Disease; depression; interstitial cystitis; vulvodynia; prostatitis; osteoarthritis; diffuse large B cell lymphoma; polymysoitis; dermatomyositis; inclusion body myositis; erosive osteoarthritis; interstitial cystitis; hepatitis; endometriosis; radiculopathy; and pyoderma gangrenosum.
17. A method of treating or preventing a disease or disorder ameliorated by the inhibition of PDE4, wherein the method comprises administering a therapeutically or prophylactically effective amount of a melt according to any one of paragraphs 10 and 11, or a pharmaceutical composition according to any one of paragraphs 13 and 14.
18. The method of paragraph 17 wherein the disease or disorder is selected from HIV; hepatitis; adult respiratory distress syndrome; bone resorption diseases; chronic obstructive pulmonary diseases; chronic pulmonary inflammatory diseases; dermatitis; inflammatory skin disease, atopic dermatitis, cystic fibrosis; septic shock; sepsis; endotoxic shock; hemodynamic shock; sepsis syndrome; post ischemic reperfusion injury; meningitis; psoriasis; fibrotic disease; cachexia; graft rejection including graft versus host disease; auto immune disease; rheumatoid spondylitis; arthritic conditions, such as rheumatoid arthritis and osteoarthritis; osteoporosis; Crohn's disease; ulcerative colitis; inflammatory bowel disease; multiple sclerosis; systemic lupus erythrematosus; erythema nodosum leprosum in leprosy; radiation damage; asthma; and hyperoxic alveolar injury.
19. A method of treating or preventing a cancer, wherein the method comprises administering a therapeutically or prophylactically effective amount of an amorphous form of a melt according to any one of paragraphs 10 and 11, or a pharmaceutical composition according to any one of paragraphs 13 and 14.
20. The method of paragraph 19 wherein the cancer is selected from multiple myeloma, malignant melanoma, malignant glioma, leukemia and a solid tumor.

## Claims

1. A process for preparing a composition comprising compound (I) which process comprises melting compound (I), together with at least one suitable excipient.

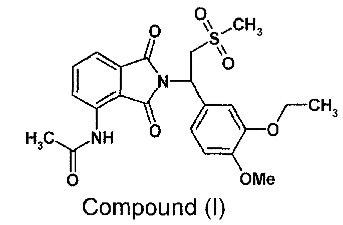

2. A process according to claim 1 wherein the composition comprises amorphous compound (I).

3. A process according to any preceding claim wherein the suitable excipient has a melting point of 50°C or more and/or a glass transition temperature of 15°C or more.

4. A process according to any preceding claim wherein the suitable excipient is selected from a polymer, a copolymer, a saccharide, an oligosaccharide, a polysaccharide, a sugar alcohol, a lipid, and a wax; preferably a polymer.

5. A process according to claim 4 wherein the polymer is selected from the group consisting of cellulose derivatives, such as hydroxypropylmethylcellulose (HPMC), carboxymethylcellulose (CMC), ethylcellulose, methylcellulose, hydroxyethylcellulose, ethylhydroxyethylcellulose, and hydroxypropylcellulose (HPC), micro-crystalline cellulose, starch, arabic gum, tragacanth gum, guar gum, alginic acid, alginates, polyvinylpyrrolidone (PVP), polyvinylacetates (PVAC), polyvinyl alcohols (PVA), polyvinyl alcohol derivatives, polymers of the acrylic acid and its salts, polyacrylamides, polymethacrylates, polymethacrylate derivatives, vinylpyrrolidone vinylacetate copolymers (copovidone), Soluton, polyalkylene glycoles, such as poly(propylene glycol) and polyethylene glycol and its derivatives such as polyethylene glycol glycerides and fatty acid esters of polyethylene glycol, co-blockpolymers of the polyethylene glycol, in particular co-block polymers of polyethylene glycol and poly(propylene glycol), co-block polymers of ethylene oxide and propylene oxide (Poloxamer, Pluronic), sucrose fatty acid esters as well as mixtures of two or more of the mentioned polymers; preferably the polymer is copovidone.

6. A process according to any preceding claim wherein compound (I) and the suitable excipient are melted at a temperature between about 50°C and about 300°C; preferably between 100°C and 250°C, more preferably between 150°C and 250°C.

7. A process according to any preceding claim further comprising extruding the product of melting compound (I), together with at least one suitable excipient.

8. A composition comprising compound (I) obtainable by a process according to any preceding claim.

9. A melt comprising compound (I).

10. A melt according to claim 9 wherein compound (I) is in an amorphous form.

11. A pharmaceutical composition comprising a composition according to claim 8, or a melt according to any one of claims 9 and 10, and a pharmaceutically acceptable excipient.

12. A pharmaceutical composition comprising an amorphous form of compound (I) and a pharmaceutically acceptable excipient.

13. A method of treating or preventing a disease or disorder ameliorated by the inhibition of TNF-[alpha] production, wherein the method comprises administering a therapeutically or prophylactically effective amount of a melt according to any one of claims 9 and 10, or a pharmaceutical composition according to any one of claims 11 and 12.

14. A method of treating or preventing a disease or disorder ameliorated by the inhibition of PDE4, wherein the method comprises administering a therapeutically or prophylactically effective amount of a melt according to any one of claims 9 and 10, or a pharmaceutical composition according to any one of claims 11 and 12.

15. A method of treating or preventing a cancer, wherein the method comprises administering a therapeutically or prophylactically effective amount of an amorphous form of a melt according to any one of claims 9 and 10, or a pharmaceutical composition according to any one of claims 11 and 12.
